# FASCICULE DE BREVET EUROPEEN

(11) **EP 1 093 778 B1**
(45) Date de publication et mention de la délivrance du brevet: **29.09.2004**
(21) Numéro de dépôt: 00402892.4
(22) Date de dépôt: 19.10.2000
(51) Int. Cl.: A61F 2/50, A61F 2/78, A61F 5/01, B29C 41/18, B29C 67/00

(54) **Procédé de fabrication d'un manchon souple pour prothèse ou orthèse et ébauche utilisée dan ce procédé**
Verfahren zum Herstellen einer biegsamen Hülse für eine Prothese oder eine Orthese und in diesem Verfahren verwendeter Rohling
Process for manufacturing a flexible sleeve for prosthesis or orthosis and blank used in this process

(30) Priorité: 22.10.1999 FR 9913202
(43) Date de publication de la demande: 25.04.2001
(73) Titulaire: Etablissements Proteor, Société Anonyme dite:, 21850 Saint Apollinarie (FR)
(72) Inventeur: Drouin, Vincent, 21200 Beaune (FR); Pierron, Olivier, 21470 Brazey en Plaine (FR)
(74) Mandataire: Jolly, Jean-Pierre

(56) Documents cités:
- EP-A- 0 207 758
- EP-A- 0 380 345
- EP-A- 0 631 765
- EP-A- 0 976 371
- WO-A-96/29033
- GB-A- 2 148 177
- US-A- 4 635 626
- US-A- 4 704 129
- US-A- 5 376 127

## Description

L'invention concerne un procédé de fabrication d'un manchon pour une prothèse ou orthèse, l'ébauche de ce manchon utilisée dans ce procédé, ainsi que le manchon ainsi obtenu.

### ETAT DE LA TECHNIQUE

Dans certains cas liés à la pathologie ou au niveau d'amputation, une prothèse externe a besoin d'une interface entre le moignon et son emboîture. Cette interface, appelée manchon, permet de protéger le moignon des sollicitations telles que chocs et frictions provenant de la marche, de la station debout ou assise, ou même de la simple mise en place de la prothèse.

Jusqu'à présent, on réalisait le manchon à partir des mesures exactes du moignon et au moyen de divers matériaux comme le caoutchouc, le cuir, le gel de silicone, le gel de polyuréthane, en mettant en oeuvre divers procédés tels que le moulage, le contre-moulage, le collage, l'injection.

Cependant, ces techniques nécessitent d'une part, un savoir-faire spécifique et, d'autre part, un temps de fabrication relativement long.

### EXPOSE SOMMAIRE DE L'INVENTION

La Demanderesse a donc conduit des recherches afin de remédier aux inconvénients qui viennent d'être mentionnés. Elle y est parvenue en utilisant une ébauche de manchon, constituée par un élément creux préfabriqué, réalisé en un matériau thermoformable souple, qui permet d'éviter les étapes actuelles de réalisation d'un patron, ainsi que de découpage, de ponçage, d'ajustage, de collage et de séchage de celui-ci.

La présente invention a par conséquent pour objet un Procédé de fabrication d'un manchon souple destiné à servir d'interface entre une prothèse ou une orthèse et une portion de membre d'un patient, caractérisé en ce qu'il comprend les étapes successives suivantes :
- on choisit une ébauche (2) de manchon, constituée d'un élément creux préfabriqué en un matériau thermoformable souple, de forme sensiblement conique ou cylindrique, comportant une extrémité fermée de forme arrondie, dont les dimensions sont adaptées à celles du moignon destiné à être équipé de la prothèse ou de l'orthèse ;
- on chauffe cette ébauche jusqu'à ce qu'elle se ramollisse,
- on enfile cette ébauche ramollie sur le moignon ou sur une reproduction de celui-ci ;
- on adapte la forme de l'ébauche ramollie à celle du moignon ou de sa reproduction ;
- on laisse refroidir l'ébauche ainsi conformée sur le moignon ou sur sa reproduction ;
- on retire du moignon ou de sa reproduction l'ébauche refroidie souple, qui constitue le manchon désiré.

L'invention a également pour objet l'ébauche de manchon souple, constituée d'un élément creux préfabriqué, en un matériau thermoformable souple, de forme sensiblement conique ou cylindrique, comportant une extrémité fermée de forme arrondie, que l'on utilise dans ce procédé.

Cette ébauche peut, par exemple, être en une mousse à cellules fermées ou ouvertes de polyoléfine ou d'éthylène vinyl acétate (EVA), ou être en tout autre matériau thermoformable utilisable industriellement pour une telle application. Cette ébauche peut également être constituée d'un mélange de deux ou plusieurs matériaux thermoformables.

L'invention concerne également le manchon souple obtenu par le procédé défini ci-dessus et qui est destiné à être interposé entre le moignon du patient, coiffé par ce manchon, et une emboîture rigide, destinée à équiper le moignon, par exemple l'emboîture rigide décrite dans US-A- 4 704 129.

Ce manchon souple reproduit parfaitement la forme du moignon du patient et lui apporte, par conséquent, un confort très supérieur à celui procuré par les manchons de la technique antérieure. En particulier, l'utilisation d'une ébauche préfabriquée par moulage, notamment par moulage par injection, que l'on adapte à la forme du moignon, sans avoir recours à un quelconque collage, élimine la présence des zones de collage des manchons de la technique antérieure, qui se révélaient à l'usage douloureuses pour le patient et pouvaient même le blesser.

D'autres caractéristiques et avantages de l'invention ressortiront de la description détaillée qui va suivre, dans laquelle on se réfèrera aux dessins schématiques annexés.

### DESCRIPTION SOMMAIRE DES FIGURES

Les figures 1a et 1b représentent schématiquement un moignon ou sa reproduction ("positif'), vu de profil et de face ;

Les figures 2a et 2b représentent schématiquement deux ébauches à partir desquelles peut être fabriqué le manchon ;

La figure 3 illustre l'opération de chauffage d'une ébauche coiffant la reproduction du moignon ;

La figure 4 est une vue en coupe de l'ébauche coiffant la reproduction du moignon, après conformation au profil de cette reproduction ;

La figure 5 illustre le démoulage du manchon refroidi ainsi réalisé ;

La figure 6 est une vue en perspective du manchon prêt à l'emploi.

### EXPOSE DETAILLE DE L'INVENTION

On peut procéder de plusieurs manières, selon que la température de ramollissement du matériau dont est faite l'ébauche est supportable par le patient ou non.

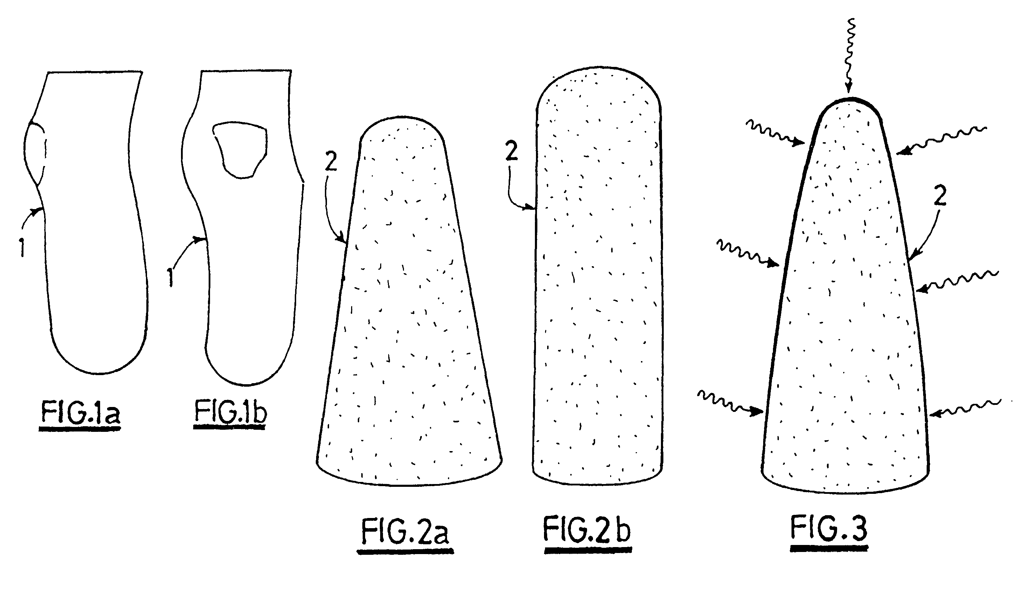

Lorsque cette température est trop élevée, on fait un moulage, puis un contre-moulage du moignon du patient, aboutissant à un moulage positif, corrigé ou non, de ce moignon, tel que celui désigné par la référence 1 sur les figures 1a et 1b.

Dans le cas où le matériau utilisé possède une température de ramollissement assez basse, il n'est pas nécessaire de recourir à un positif et on évite ainsi avantageusement toute la phase de prise d'empreintes et de rectification du positif.

On choisit ensuite une ébauche creuse 2 du manchon, en un matériau thermoformable souple, préfabriquée par moulage, par exemple de forme grossièrement conique (figure 2a) ou cylindrique (figure 2b), comportant une extrémité fermée de forme arrondie, dont les dimensions sont adaptées à celles du moignon du patient. On effectue le choix de l'ébauche en mesurant le périmètre du moignon ou du positif à une distance déterminée de son extrémité et en la comparant à la dimension correspondante de l'ébauche à un emplacement correspondant.

On chauffe alors l'ébauche 2 retenue (figure 3) par exemple au four, ou à l'aide d'un canon à air chaud, ou par tout autre moyen connu dans la technique, et, lorsqu'elle est suffisamment ramollie, on l'enfile sur le moignon ou sur le positif 1 correspondant à celui-ci.

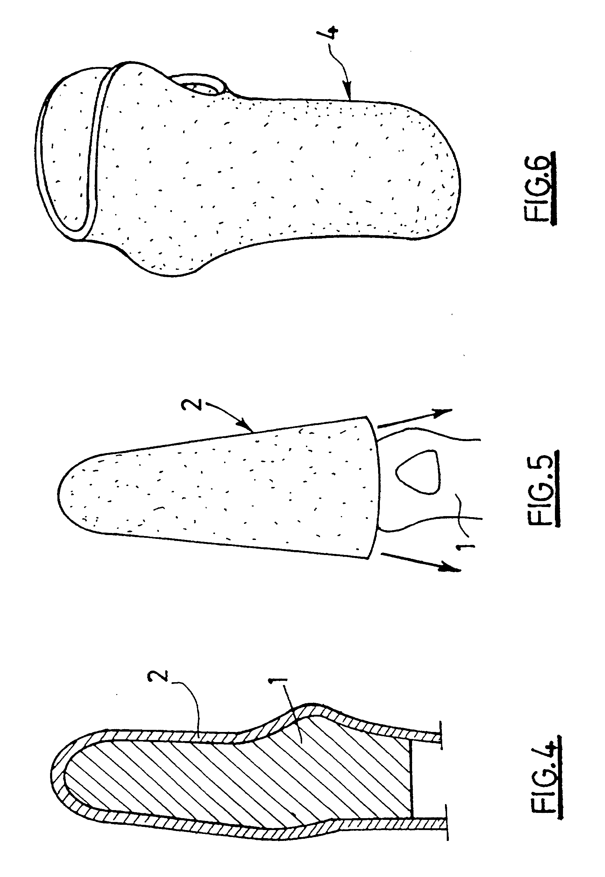

Du fait du ramollissement de l'ébauche 2, on peut alors lui faire épouser étroitement, la forme du moignon ou du positif 1 (figure 4), en exerçant une pression sur la surface externe du manchon ou une dépression à l'intérieur de celui-ci, l'ébauche 2 se conformant ainsi parfaitement au profil et aux dimensions du moignon ou du positif utilisé.

Après refroidissement de l'ébauche, il suffit de la retirer du moignon ou du positif 1 (figure 5), pour obtenir un manchon souple 4 (figure 6), dont la forme et les dimensions correspondent exactement à celles du moignon du patient. Les découpes supérieures du manchon 4 sont réalisées ensuite à l'aide d'un outil tranchant et les bords du manchon sont de préférence poncés, pour éviter de blesser le patient, sur le moignon duquel le manchon peut être mis en place sans autre adaptation.

L'invention apporte donc un moyen particulièrement simple, facile à mettre en oeuvre et peu coûteux, pour réaliser des manchons qui épousent fidèlement la forme et les dimensions du moignon du patient à qui ce manchon est destiné.

## Revendications

1. Procédé de fabrication d'un manchon souple destiné à servir d'interface entre une prothèse ou une orthèse et une portion de membre d'un patient, **caractérisé en ce qu'**il comprend les étapes successives suivantes :
- on choisit une ébauche (2) de manchon, constituée d'un élément creux préfabriqué en un matériau thermoformable souple, de forme sensiblement conique ou cylindrique, comportant une extrémité fermée de forme arrondie, dont les dimensions sont adaptées à celles du moignon destiné à être équipé de la prothèse ou de l'orthèse ;
- on chauffe cette ébauche (2) jusqu'à ce qu'elle se ramollisse,
- on enfile cette ébauche ramollie sur le moignon ou sur une reproduction (1) de celui-ci ;
- on adapte la forme de l'ébauche ramollie (2) à celle du moignon ou de sa reproduction (1) ;
- on laisse refroidir l'ébauche (2) ainsi conformée sur le moignon ou sur sa reproduction ;
- on retire du moignon ou de sa reproduction l'ébauche refroidie souple, qui constitue le manchon (4) désiré.

2. Procédé selon la revendication 1, **caractérisé en ce que** l'ébauche souple (2) est en une mousse de polyoléfine ou d'éthylène vinyl acétate, seuls ou en mélange avec d'autres matériaux thermoformables.

3. Procédé selon l'une des revendications 1 et 2, **caractérisé en ce que** l'ébauche souple (2) ramollie est mise à la forme du moignon ou de la reproduction (1) de celui-ci en exerçant une pression à l'extérieur de cette ébauche.

4. Procédé selon l'une des revendications 1 et 2, **caractérisé en ce que** l'ébauche souple (2) ramollie est mise à la forme du moignon ou de la reproduction (1) de celui-ci en exerçant une dépression à l'intérieur de cette ébauche.

5. Ebauche (2) de manchon souple utilisable dans le procédé selon l'une des revendications 1 à 4, **caractérisée en ce qu'**elle est constituée d'un élément creux préfabriqué, en un matériau thermoformable souple, de forme sensiblement conique ou cylindrique, comportant une extrémité fermée de forme arrondie.

6. Ebauche selon la revendication 5, **caractérisée en ce qu'**elle est en une mousse de polyoléfine ou d'éthylène vinyl acétate, seuls ou en mélange avec d'autres matériaux thermoformables.

## Patentansprüche

1. Verfahren zum Herstellen einer nachgiebigen Manschette, die dazu vorgesehen ist, als Verbindungsstelle zwischen einer Prothese oder einer Orthese und einem Gliedabschnitt eines Patienten zu dienen,
**dadurch gekennzeichnet, dass** es die folgenden aufeinanderfolgenden Schritte enthält:
Wählen eines Manschettenrohlings (2), der aus einem hohlen vorgefertigten Element aus einem thermisch formbaren nachgiebigen Material besteht, mit einer im Wesentlichen konischen oder zylindrischen Form, die ein geschlossenes Ende mit einer runden Form aufweist, dessen Abmessungen an die des Stumpfes angepasst sind, der dazu vorgesehen ist, mit der Prothese oder der Orthese versehen zu werden;
Erwärmen des Rohlings (2), bis er weich wird;
Überziehen des erweichten Rohlings (2) auf den Stumpf oder auf dessen Nachbildung (1);
Anpassen der Form des erweichten Rohlings (2) an die des Stumpfes oder seiner Nachbildung (1);
Abkühlenlassen des so angepassten Rohlings (2) auf dem Stumpf oder auf seiner Nachbildung (1);
Abziehen des abgekühlten nachgiebigen Rohlings (2), der die gewünschte Manschette (4) bildet, von dem Stumpf oder von seiner Nachbildung (1).

2. Verfahren nach Anspruch 1, **dadurch gekennzeichnet, dass** der nachgiebige Rohling (2) aus einem Schaum aus Polyolefin oder Ethylenvinylacetat für sich allein oder in Mischung mit anderen thermisch formbaren Materialien ist.

3. Verfahren nach Anspruch 1 oder 2, **dadurch gekennzeichnet, dass** der nachgiebige erweichte Rohling (2) in die Form des Stumpfes oder seiner Nachbildung (1) gebracht wird, indem ein Druck auf das Äußere des Rohlings ausgeübt wird.

4. Verfahren nach Anspruch 1 oder 2, **dadurch gekennzeichnet, dass** der nachgiebige erweichte Rohling (2) in die Form des Stumpfes oder seiner Nachbildung (1) gebracht wird, indem ein Unterdruck auf das Innere des Rohlings ausgeübt wird.

5. Nachgiebiger Manschettenrohling (2), der in dem Verfahren nach einem der Ansprüche 1 bis 4 verwendet werden kann, **dadurch gekennzeichnet, dass** er aus einem hohlen vorgefertigten Element aus einem thermisch formbaren nachgiebigen Material besteht, mit einer im Wesentlichen konischen oder zylindrischen Form, die ein geschlossenes Ende mit einer runden Form aufweist.

6. Rohling nach Anspruch 5, **dadurch gekennzeichnet, dass** er aus einem Schaum aus Polyolefin oder Ethylenvinylacetat für sich allein oder in Mischung mit anderen thermisch formbaren Materialien ist.

## Claims

1. A process for manufacturing a flexible sleeve designed to serve as an interface between a prosthesis or an orthosis and a limb portion of a patient, **characterised in that** it comprises the following successive stages:
- a sleeve blank (2) is selected, consisting of a prefabricated hollow element of a flexible thermoformable material, substantially conical or cylindrical in shape, comprising a closed end of rounded shape, the dimensions of which are conformed to those of the stump intended to be fitted with the prosthesis or the orthosis;
- said blank (2) is heated until it softens;
- said softened blank is slipped onto the stump or onto a replica (1) thereof;
- the shape of the softened blank (2) is adapted to that of the stump or of the replica (1) thereof;
- the blank (2) thus conformed is left to cool on the stump or on the replica thereof;
- the flexible cooled blank, which constitutes the desired sleeve (4), is removed from the stump or the replica thereof.

2. A process according to claim 1, **characterised in that** the flexible blank (2) is made of a polyolefin or ethylene vinyl acetate foam, alone or mixed with other thermoformable materials.

3. A process according to either one of claims 1 and 2, **characterised in that** the softened flexible blank (2) is adjusted to the shape of the stump or of the replica (1) thereof by exerting pressure on the outside of said blank.

4. A process according to either one of claims 1 and 2, **characterised in that** the softened flexible blank (2) is adjusted to the shape of the stump or of the replica (1) thereof by reducing the pressure inside said blank.

5. A flexible sleeve blank (2) which may be used in the process according to any one of claims 1 to 4, **characterised in that** it consists of a prefabricated hollow element of a flexible thermoformable material, substantially conical or cylindrical in shape, comprising a closed end of rounded shape.

6. A blank according to claim 5, **characterised in that** it is made of a polyolefin or ethylene vinyl acetate foam, alone or mixed with other thermoformable materials.
